# EUROPEAN PATENT APPLICATION

(11) **EP 4 763 040 A1**
(43) Date of publication of application: **24.06.2026**
(21) Application number: 25220809.5
(22) Date of filing: 04.12.2025
(51) Int. Cl.: A61B 1/00, A61B 1/24, A61C 9/00

(54) **WIRELESS INTRAORAL SCANNER AND INTRAORAL SCAN IMAGE FORMING APPARATUS HAVING THE SAME**

(30) Priority: 18.12.2024 KR 20240189587
(71) Applicant: Huvitz Co., Ltd., Anyang-si, Gyeonggi-do 14055 (KR)
(72) Inventor: CHO, Min Soo, 14055 Anyang-si (KR); LEE, Yong Jin, 14055 Anyang-si (KR); LEE, Weon Joon, 14055 Anyang-si (KR)
(74) Representative: Henkel & Partner mbB

(57) **Abstract**

A wireless intraoral scanner and an intraoral scan image forming apparatus including the same are disclosed. The intraoral scan image forming apparatus comprises: an intraoral scanner (10) that optically scans and captures intraoral structures to obtain two-dimensional shape image data of the intraoral structures; a data processor (22) that outputs a control signal for the intraoral scanner (10) and forms a three-dimensional shape image of the intraoral structures from the two-dimensional shape image data of the intraoral structures obtained from the intraoral scanner (10); a wireless auxiliary device (30) that supplies power for driving the intraoral scanner (10) to the intraoral scanner (10) through a wired signal line (19), receives a wired signal transmitted from the intraoral scanner (10) through the wired signal line (19), converts it into a wireless signal and transmits it to the data processor (22), receives a wireless signal transmitted wirelessly from the data processor (22), converts it into a wired signal and transmits it to the intraoral scanner (10); and a wireless transmission/reception device (80) that receives the wireless signal transmitted from the wireless auxiliary device (30), converts it into a wired signal and transmits it to the data processor (22), receives a wired signal transmitted from the data processor (22), converts it into a wireless signal and transmits it to the wireless auxiliary device (30).

## Description

### CROSS-REFERENCE TO RELATED APPLICATION

This application claims benefit of priority to Korean Patent Application No. 10-2024-0189587 filed on December 18, 2024, the entire contents of which are incorporated herein by reference.

### FIELD OF THE DISCLOSURE

The present invention relates to an intraoral scanner, and more particularly, to an intraoral scanner that is inserted into a dental patient's oral cavity to acquire three-dimensional shape information of intraoral structures, and specifically to a wireless intraoral scanner and an intraoral scan image forming apparatus having the same.

### BACKGROUND

In general, at dental clinics and the like, the shape of a patient's teeth or gums in the oral cavity is examined, and based on this, the patient's oral condition is diagnosed or prosthetics are manufactured. Recently, optical intraoral scan image forming apparatuses have been used that optically scan and capture the interior of a patient's oral cavity without physical contact to obtain shape images of intraoral structures.

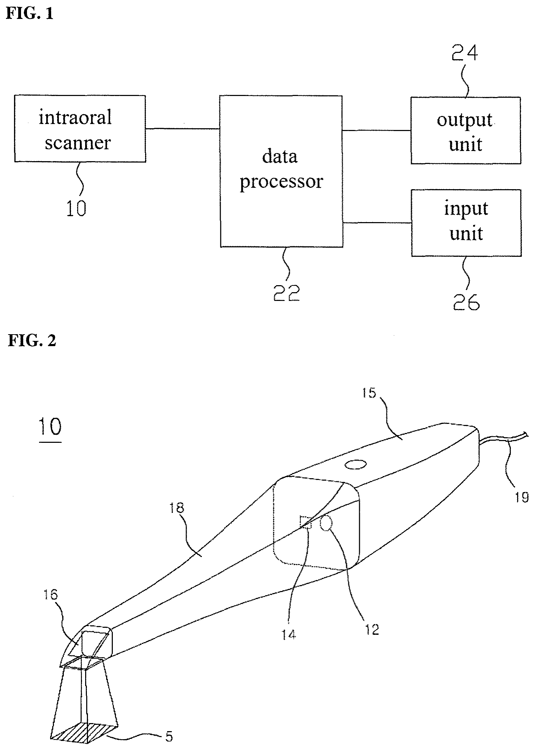

FIG. 1 is a diagram illustrating the configuration of a conventional intraoral scan image forming apparatus. As shown in FIG. 1, a conventional intraoral scan image forming apparatus includes an intraoral scanner 10, a data processor 22, an output unit 24, and an input unit 26.

The intraoral scanner 10 is a device that optically scans and captures a patient's intraoral structures, such as teeth and gums, to obtain two-dimensional shape image data of the intraoral structures. FIG. 2 is a perspective view for explaining the configuration of a conventional intraoral scanner 10. As shown in FIG. 2, the conventional intraoral scanner 10 includes a measurement light source 12 that irradiates measurement light onto the intraoral structures, a shape detector 14 that detects reflected light formed by the reflection of the measurement light from the intraoral structures, an intraoral scanner body 15 that accommodates the measurement light source 12 and the shape detector 14, and an intraoral tip 18. The intraoral tip 18 is equipped with a reflection mirror 16 that reflects the measurement light generated from the measurement light source 12 to irradiate it onto the intraoral structures and reflects the reflected light from the intraoral structures to guide it to the shape detector 14. In FIG. 2, reference numeral 19 denotes a wired signal line that supplies power to the intraoral scanner 10 and transmits data and/or control signals between the intraoral scanner 10 and the data processor 22.

A user holds the intraoral scanner body 15 by hand, positions the intraoral tip 18 inside the patient's oral cavity, irradiates measurement light onto a predetermined area 5 inside the oral cavity using the measurement light source 12, and detects the reflected light generated by the reflection of the measurement light from the area 5 using the shape detector 14 to obtain two-dimensional shape image data of the intraoral structures.

Referring back to FIG. 1, the data processor 22 processes the two-dimensional shape image data of the intraoral structures obtained from the intraoral scanner 10 to generate a three-dimensional shape image of the intraoral structures necessary for the patient's examination or treatment. The output unit 24 is a device that displays on a screen the processing of the intraoral scan image performed by the data processor 22, the shape image of the intraoral structures obtained thereby, and the like. The input unit 26 is a device for inputting user commands to the data processor 22.

Conventionally, since the intraoral scanner 10 that scans the shape of the intraoral structures and the data processor 22 that processes the shape image data obtained from the intraoral scanner 10 are connected by the wired signal line 19, there is inconvenience in that the user cannot freely use the intraoral scanner 10.

To improve such disadvantages, wireless intraoral scanners have also been used, in which a wireless data transmission/reception device such as Bluetooth or Wi-Fi and a power source for driving the intraoral scanner 10, i.e., a battery, are mounted inside the intraoral scanner 10, and communication between the intraoral scanner 10 and the data processor 22 is performed wirelessly.

However, a conventional wireless intraoral scanner 10 including a wireless data transmission/reception device and a battery is not only inconvenient to use due to constraints on size and weight, but also has disadvantages in that the capacity of the mounted battery is small and the usage time is limited.

### [Prior Art Documents]

(Patent Document 1) U.S. Patent No. 9,629,551
(Patent Document 2) U.S. Patent No. 10,064,553
(Patent Document 3) U.S. Patent No. 10,695,151
(Patent Document 4) U.S. Patent No. 11,076,146

### SUMMARY OF THE DISCLOSURE

### TECHNICAL OBJECTS

An object of the present invention is to provide a wireless intraoral scanner that is wirelessly connected to a data processor such as a computer, allowing free use, and has relatively fewer constraints on the size and weight of a wireless transmission/reception device, battery, and the like, and an intraoral scan image forming apparatus having the same.

### TECHNICAL SOLUTION

To achieve the above object, the present invention provides a wireless intraoral scan image forming apparatus comprising: an intraoral scanner configured to optically scan and capture intraoral structures to obtain two-dimensional shape image data of the intraoral structures; a data processor configured to output a control signal for driving the intraoral scanner and form a three-dimensional shape image of the intraoral structures from the two-dimensional shape image data of the intraoral structures obtained from the intraoral scanner ; a wireless auxiliary device configured to supply power for driving the intraoral scanner to the intraoral scanner through a wired signal line, receive a wired signal transmitted from the intraoral scanner through the wired signal line, convert the wired signal into a wireless signal and transmit the wireless signal to the data processor, receive a wireless signal transmitted wirelessly from the data processor, convert the wireless signal into a wired signal and transmit the wired signal to the intraoral scanner; and a wireless transmission/reception device configured to receive the wireless signal transmitted from the wireless auxiliary device, convert the wireless signal into a wired signal and transmits the wired signal to the data processor, receive a wired signal transmitted from the data processor, convert the wired signal into a wireless signal and transmit the wireless signal to the wireless auxiliary device.

In addition, the present invention provides a wireless intraoral scanner comprising the intraoral scanner and the wireless auxiliary device.

### EFFECTS OF THE DISCLOSURE

The wireless intraoral scanner according to the present invention is wirelessly connected to a data processor such as a computer, allowing free movement, and have the advantage of relatively fewer constraints on the size and weight of a wireless transmission/reception device, battery, and the like.

### BRIEF DESCRIPTION OF THE DRAWINGS

FIG. 1 is a diagram illustrating the configuration of a conventional intraoral scan image forming apparatus.
FIG. 2 is a perspective view for explaining the configuration of a conventional intraoral scanner.
FIG. 3 is a diagram illustrating the configuration of a wireless intraoral scan image forming apparatus according to an embodiment of the present invention.
FIG. 4 is a diagram illustrating the configuration of an intraoral scanner that can be used in the present invention.
FIG. 5 is a diagram illustrating the configuration of a wireless auxiliary device that can be used in the present invention.
FIG. 6 is a diagram illustrating the configuration of a wireless transmission/reception device that can be used in the present invention.
FIG. 7 is a diagram illustrating the configuration of a wireless intraoral scan image forming apparatus according to another embodiment of the present invention.

### DETAILED DESCRIPTION OF THE PREFERRED EMBODIMENTS

Hereinafter, the present invention will be described in detail with reference to the accompanying drawings. In the accompanying drawings, the same reference numerals are assigned to components that perform the same or similar functions as in the prior art.

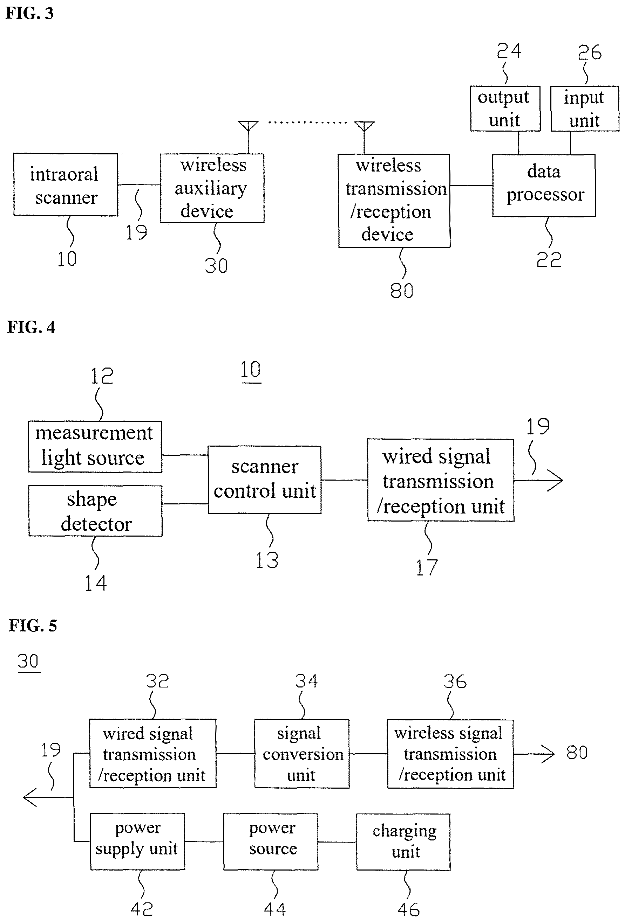

FIG. 3 is a diagram illustrating the configuration of a wireless intraoral scan image forming apparatus according to an embodiment of the present invention. As shown in FIG. 3, the wireless intraoral scan image forming apparatus according to the present invention comprises an intraoral scanner 10, a wireless auxiliary device 30, a wireless transmission/reception device 80, and a data processor 22, and may further include an output unit 24 and an input unit 26 as needed. The wireless intraoral scanner according to the present invention comprises the intraoral scanner 10 and the wireless auxiliary device 30.

The intraoral scanner 10 is a device that optically scans and captures a patient's intraoral structures, such as teeth and gums, to obtain two-dimensional shape image data of the intraoral structures. For example, the intraoral scanner 10 is a conventional wired intraoral scanner that is not equipped with a wireless transmission/reception device such as Bluetooth or Wi-Fi device and/or a separate power source for driving the intraoral scanner 10, i.e., a separate battery.

FIG. 4 is a block diagram illustrating the configuration of an intraoral scanner 10 that can be used in the present invention. Referring to FIGS. 2 and 4, the intraoral scanner 10 that can be used in the present invention comprises a measurement light source 12 that irradiates measurement light onto the intraoral structures, a shape detector 14 that detects reflected light formed by the reflection of the measurement light from the intraoral structures, a scanner control unit 13 that outputs a control signal for controlling the measurement light source 12 and obtains two-dimensional shape image data of the intraoral structures from the signal of the reflected light detected by the shape detector 14, and a wired signal transmission/reception unit 17 that transmits the control signal and the two-dimensional shape image data through a wired signal line 19.

In addition, as shown in FIG. 2, the intraoral scanner 10 may include an intraoral scanner body 15 that accommodates the measurement light source 12 and the shape detector 14, and an intraoral tip 18 equipped with a reflection mirror 16 that reflects the measurement light generated from the measurement light source 12 to irradiate it onto the intraoral structures and reflects the reflected light from the intraoral structures to guide it to the shape detector 14.

A user holds the intraoral scanner body 15 by hand, positions the intraoral tip 18 inside the patient's oral cavity, irradiates measurement light onto a predetermined area 5 inside the oral cavity using the measurement light source 12, and detects the reflected light generated by the reflection of the measurement light from the area 5 using the shape detector 14 to obtain two-dimensional shape image data of the intraoral structures. The measurement light may be a structured light generated by methods such as Digital Light Processing (DLP) or color-coded pattern formation. The structured light thus generated is irradiated onto a target, i.e., a predetermined area 5 inside the oral cavity, and then the reflected light from the target is detected by the shape detector 14, i.e., an internal camera sensor. Specifically, using the scanner control unit 13 of the intraoral scanner 10, a two-dimensional image (2D image) of the target is obtained from the signal of the shape detector 14, i.e., the camera sensor, in accordance with a predetermined synchronization signal, and transmitted to the wireless auxiliary device 30.

One end of the intraoral scanner 10, preferably one end of the intraoral scanner body 15, is provided with a wired signal line 19 that supplies power to the intraoral scanner 10 and transmits signals, specifically data signals and/or control signals, between the intraoral scanner 10 and the wireless auxiliary device 30 (see FIG. 2). The wired signal line 19 may transmit power and signals using a wired communication method such as a USB 3.1 interface (I/F).

The data processor 22 outputs a control signal for driving the intraoral scanner 10 and forms (reconstructs) a three-dimensional shape image of the intraoral structures necessary for the patient's examination or treatment from the two-dimensional shape image data of the intraoral structures obtained from the intraoral scanner 10. Since the shape of the two-dimensional image obtained from the intraoral scanner 10 changes according to the three-dimensional shape of the intraoral structures, the obtained two-dimensional image can be analyzed (converted) using the data processor 22 to obtain the three-dimensional shape of the intraoral structures. The data processor 22 may be a computer equipped with application software (application S/W) capable of processing shape image data (i.e., performing analysis, movement, comparison, registration, etc. of shape image data), for example, a personal computer (PC) or a notebook computer.

The wireless auxiliary device 30 supplies power for driving the intraoral scanner 10 to the intraoral scanner 10 through the wired signal line 19. FIG. 5 is a block diagram illustrating the configuration of a wireless auxiliary device 30 that can be used in the present invention. As shown in FIG. 5, the wireless auxiliary device 30 comprises a power source 44 in which power for driving the intraoral scanner 10 is stored, and a power supply unit 42 that supplies the power from the power source 44 to the intraoral scanner 10.

In the present invention, the wireless auxiliary device 30 supplies the power from the power source 44 to the intraoral scanner 10 through the wired signal line 19, thereby serving as the power source for the intraoral scanner 10. The power source 44 may be, for example, a secondary battery. The power supply unit 42 may transmit the power from the power source 44 to the intraoral scanner 10 using a USB power delivery protocol (PD). At this time, depending on the specifications of the intraoral scanner 10, the supplied current can be varied to an appropriate rated current for each intraoral scanner 10 through PD negotiation. Thereby, a flexible rated current supply system can be established, allowing the wireless auxiliary device 30 to be used for intraoral scanners 10 of various specifications.

As needed, the wireless auxiliary device 30 may further include a charging unit 46 for charging the power source 44. The charging unit 46 may be connected to a conventional power supply device such as a separate wireless charging battery pack or charger to charge the power source 44. Even during use of the wireless auxiliary device 30, the wireless auxiliary device 30 can be charged by using the charging unit 46, and also power can be supplied to the intraoral scanner 10 thereby improving usability.

Since the wireless auxiliary device 30 uses the power from the built-in power source 44, i.e., the built-in battery, a separate wired connection for receiving external power is not necessary. However, as needed, when connecting an auxiliary battery, external charger, etc., to the wireless auxiliary device 30, it is possible to use external power while simultaneously charging the power source 44. When the charge level of the power source 44 is insufficient, charging can be done via wired or wireless methods. The wired charging method can use the same USB-Type C PPS protocol as smartphone charging, and the wireless charging method can charge the power source 44 while using the intraoral scanner 10 by using a wireless battery pack.

In addition, the wireless auxiliary device 30 receives a wired signal transmitted from the intraoral scanner 10 through the wired signal line 19, converts it into a wireless signal and transmits it to the data processor 22. The wireless auxiliary device 30 also receives a wireless signal transmitted wirelessly from the data processor 22, converts it into a wired signal and transmits it to the intraoral scanner 10.

The two-dimensional shape image (2D image) data received from the intraoral scanner 10 is transmitted wirelessly to the data processor 22 according to a predetermined protocol and flow. As shown in FIG. 5, preferably, the wireless auxiliary device 30 comprises: (a) a wired signal transmission/reception unit 32 that receives two-dimensional shape image data transmitted from the intraoral scanner 10 through the wired signal line 19 and transmits a control signal transmitted from the data processor 22 to the intraoral scanner 10 through the wired signal line 19; (b) a signal conversion unit 34 that converts the two-dimensional shape image data transmitted from the wired signal transmission/reception unit 32 into a wireless signal (for transmission via wireless communication) and converts the control signal transmitted as a wireless signal from the data processor 22 into a wired signal; and (c) a wireless signal transmission/reception unit 36 that wirelessly transmits the two-dimensional shape image data in the form of a wireless signal converted by the signal conversion unit 34 to the data processor 22 and receives the control signal transmitted as a wireless signal from the data processor 22.

Here, in converting the two-dimensional shape image data transmitted via wired connection into two-dimensional shape image data in wireless signal form, the signal conversion unit 34 of the wireless auxiliary device 30 may reduce data capacity through a data compression algorithm to ensure high-speed wireless communication, generate a checksum for verifying data integrity, and generate final transmission data using the compressed data and the checksum.

The intraoral scanner 10 and the wireless auxiliary device 30 are connected by the wired signal line 19 to transmit signals, i.e., communicate. Here, the transmitted signals include data and/or control signals. The communication between the intraoral scanner 10 and the wireless auxiliary device 30 may be performed using a conventional wired communication protocol such as a USB communication interface (I/F).

The wireless auxiliary device 30 used in the present invention is connected to the intraoral scanner 10 by the wired signal line 19 to transmit power and signals. Specifically, it supplies power for driving the intraoral scanner 10 to the intraoral scanner 10 through the wired signal line 19, converts the wired two-dimensional shape image data transmitted from the intraoral scanner 10 through the wired signal line 19 into wireless data, and wirelessly transmits it to the data processor 22.

The wireless auxiliary device 30 is a separate device not built into the intraoral scanner 10, and the size of the wireless auxiliary device 30 may be the size of a conventional portable battery, and it may be connected to the intraoral scanner 10 using a conventional wired connection cable 19.

The wireless transmission/reception device 80 receives the wireless signal transmitted from the wireless auxiliary device 30, converts it into a wired signal and transmits it to the data processor 22. The wireless transmission/reception device 80 also receives a wired signal from the data processor 22, converts it into a wireless signal and transmits it to the wireless auxiliary device 30.

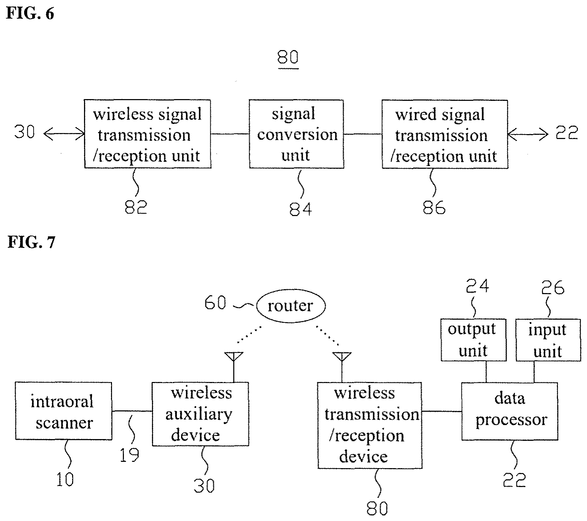

FIG. 6 is a block diagram illustrating the configuration of a wireless transmission/reception device 80 that can be used in the present invention. As shown in FIG. 6, the wireless transmission/reception device 80 comprises: (a) a wireless signal transmission/reception unit 82 that receives two-dimensional shape image data transmitted wirelessly from the wireless auxiliary device 30 and wirelessly transmits the control signal transmitted from the data processor 22 to the wireless auxiliary device 30; (b) a signal conversion unit 84 that converts the two-dimensional shape image data transmitted from the wireless signal transmission/reception unit 82 into a wired signal and converts the control signal transmitted as a wired signal from the data processor 22 into a wireless signal; and (c) a wired signal transmission/reception unit 86 that transmits the two-dimensional shape image data in the form of a wired signal converted by the signal conversion unit 84 to the data processor 22 via wired connection and receives the control signal transmitted as a wired signal from the data processor 22.

The signal conversion unit 84 of the wireless transmission/reception device 80 verifies the integrity of the transmitted signal, i.e., the compressed two-dimensional shape image data, using the checksum, and converts the wireless two-dimensional shape image data back to the original wired data by using a predetermined algorithm.

The communication between the wireless auxiliary device 30 and the wireless transmission/reception device 80 may be performed using a conventional wireless communication protocol such as Bluetooth or Wi-Fi.

Referring back to FIG. 3, the output unit 24 is a device that displays on a screen the processing of the intraoral scan image performed by the data processor 22, the three-dimensional shape image of the intraoral structures obtained thereby, and the like. The output unit 24 is, for example, a conventional display device such as a liquid crystal display (LCD) device.

The input unit 26 is a device for inputting user commands. The input unit 26 is, for example, a device for inputting user operation commands such as movement or rotation of the shape image of the intraoral structures to a user interface displayed on the output unit 24. The input unit 26 is, for example, a conventional input device such as a keyboard or mouse.

The wireless intraoral scanner according to the present invention comprises: an intraoral scanner 10 that optically scans and captures intraoral structures to obtain two-dimensional shape image data of the intraoral structures; and a wireless auxiliary device 30 that supplies power for driving the intraoral scanner 10 to the intraoral scanner 10 through a wired signal line 19, receives a wired signal transmitted from the intraoral scanner 10 through the wired signal line 19, converts it into a wireless signal and transmits it to a data processor 22, receives a wireless signal transmitted wirelessly from the data processor 22, converts it into a wired signal and transmits it to the intraoral scanner 10. Here, the data processor 22 outputs the control signal for driving the intraoral scanner 10, and forms a three-dimensional shape image of the intraoral structures from the two-dimensional shape image data of the intraoral structures obtained from the intraoral scanner 10.

FIG. 7 is a diagram illustrating the configuration of a wireless intraoral scan image forming apparatus according to another embodiment of the present invention. The wireless intraoral scan image forming apparatus shown in FIG. 7 differs from the wireless intraoral scan image forming apparatus shown in FIG. 3 in that the wireless connection between the wireless auxiliary device 30 and the wireless transmission/reception device 80 is not made directly but performed through a conventional router 60.

As shown in FIG. 7, when wireless communication is performed through a general-purpose wireless router 60, the physical distance between the intraoral scanner according to the present invention, i.e., the physical distance between "the intraoral scanner 10 and the wireless auxiliary device 30" and "the wireless transmission/reception device 80 and the data processor 22" can be increased. For example, "the wireless transmission/reception device 80 and the data processor 22" may be installed indoors in a hospital or the like, and a user may examine a patient outdoors using the intraoral scanner according to the present invention, i.e., "the intraoral scanner 10 and the wireless auxiliary device 30".

The router 60 may be a general-purpose Wi-Fi router, and the wireless communication between the wireless auxiliary device 30 and the wireless transmission/reception device 80 may be performed through a Wi-Fi communication network.

According to the present invention, without mounting the wireless auxiliary device 30 inside the intraoral scanner 10, by connecting the wireless auxiliary device 30 to the outside of a conventional wired intraoral scanner 10, power and signals are supplied to the intraoral scanner 10 via wired connection, and data from the intraoral scanner 10 is wirelessly transmitted to the data processor 22, thereby improving user convenience.

According to the present invention, a user can carry the wireless auxiliary device 30 in the user's pocket or position it near the patient, obtain intraoral scan image data of the patient using the intraoral scanner 10 wiredly connected to the wireless auxiliary device 30, and generate a three-dimensional image of the patient's oral cavity from the patient's intraoral scan image data using the data processor 22 wirelessly connected to the wireless auxiliary device 30.

Although the present invention has been described with reference to the accompanying drawings and exemplary embodiments, the present invention is not limited to the contents shown in the drawings and the above-described embodiments. For the purpose of understanding, reference numerals are indicated in the following claims, but the scope of the following claims is not limited to the reference numerals and the contents shown in the drawings, and should be interpreted to encompass all modifications, equivalents, and functions of the exemplary embodiments.

## Claims

1. A wireless intraoral scan image forming apparatus comprising:
an intraoral scanner (10) configured to optically scan and capture intraoral structures to obtain two-dimensional shape image data of the intraoral structures;
a data processor (22) configured to output a control signal for driving the intraoral scanner (10) and form a three-dimensional shape image of the intraoral structures from the two-dimensional shape image data of the intraoral structures obtained from the intraoral scanner (10);
a wireless auxiliary device (30) configured to supply power for driving the intraoral scanner (10) to the intraoral scanner (10) through a wired signal line (19), receive a wired signal transmitted from the intraoral scanner (10) through the wired signal line (19), convert the wired signal into a wireless signal and transmit the wireless signal to the data processor (22), receive a wireless signal transmitted wirelessly from the data processor (22), convert the wireless signal into a wired signal and transmit the wired signal to the intraoral scanner (10); and
a wireless transmission/reception device (80) configured to receive the wireless signal transmitted from the wireless auxiliary device (30), convert the wireless signal into a wired signal and transmits the wired signal to the data processor (22), receive a wired signal transmitted from the data processor (22), convert the wired signal into a wireless signal and transmit the wireless signal to the wireless auxiliary device (30).

2. The wireless intraoral scan image forming apparatus of claim 1, wherein the intraoral scanner (10) is not equipped with a wireless transmission/reception device or a power source for driving the intraoral scanner (10).

3. The wireless intraoral scan image forming apparatus of claim 1, wherein the wireless auxiliary device (30) comprises: a power source (44) in which power for driving the intraoral scanner (10) is stored; and a power supply unit (42) configured to supply the power from the power source (44) to the intraoral scanner (10).

4. The wireless intraoral scan image forming apparatus of claim 3, wherein the power supply unit (42) transmits the power from the power source (44) to the intraoral scanner (10) by using a USB power delivery protocol.

5. The wireless intraoral scan image forming apparatus of claim 3, wherein the wireless auxiliary device (30) further comprises a charging unit (46) for charging the power source (44).

6. The wireless intraoral scan image forming apparatus of claim 1, wherein the wireless auxiliary device (30) comprises:
(a) a wired signal transmission/reception unit (32) configured to receive two-dimensional shape image data transmitted from the intraoral scanner (10) through the wired signal line (19) and transmit the control signal transmitted from the data processor (22) to the intraoral scanner (10) through the wired signal line (19);
(b) a signal conversion unit (34) configured to convert the two-dimensional shape image data transmitted from the wired signal transmission/reception unit (32) into a wireless signal and convert the control signal transmitted as a wireless signal from the data processor (22) into a wired signal; and
(c) a wireless signal transmission/reception unit (36) configured to wirelessly transmit the two-dimensional shape image data in the form of the wireless signal converted by the signal conversion unit (34) to the data processor (22) and receive the control signal transmitted as the wireless signal from the data processor (22).

7. The wireless intraoral scan image forming apparatus of claim 1, wherein the wireless transmission/reception device (80) comprises:
(a) a wireless signal transmission/reception unit (82) configured to receive two-dimensional shape image data transmitted wirelessly from the wireless auxiliary device (30) and wirelessly transmit the control signal transmitted from the data processor (22) to the wireless auxiliary device (30);
(b) a signal conversion unit (84) configured to convert the two-dimensional shape image data transmitted from the wireless signal transmission/reception unit (82) into a wired signal and convert the control signal transmitted as a wired signal from the data processor (22) into a wireless signal; and
(c) a wired signal transmission/reception unit (86) configured to transmit the two-dimensional shape image data in the form of a wired signal converted by the signal conversion unit (84) to the data processor (22) via wired connection and receive the control signal transmitted as a wired signal from the data processor (22).

8. The wireless intraoral scan image forming apparatus of claim 1, wherein a wireless connection between the wireless auxiliary device (30) and the wireless transmission/reception device (80) is performed through a router (60).

9. A wireless intraoral scanner comprising:
an intraoral scanner (10) configured to optically scan and capture intraoral structures to obtain two-dimensional shape image data of the intraoral structures; and
a wireless auxiliary device (30) configured to supply power for driving the intraoral scanner (10) to the intraoral scanner (10) through a wired signal line (19), receive a wired signal transmitted from the intraoral scanner (10) through the wired signal line (19), convert the wired signal into a wireless signal and transmit the wireless signal to a data processor (22), receive a wireless signal transmitted wirelessly from the data processor (22), convert the wireless signal into a wired signal and transmit the wired signal to the intraoral scanner (10),
wherein the data processor (22) is configured to output a control signal for driving the intraoral scanner (10), and form a three-dimensional shape image of the intraoral structures from the two-dimensional shape image data of the intraoral structures obtained from the intraoral scanner (10).
